# EUROPEAN PATENT SPECIFICATION

(11) **EP 0 533 378 B1**
(45) Date of publication and mention of the grant of the patent: **04.12.1996**
(21) Application number: 92308031.1
(22) Date of filing: 04.09.1992
(51) Int. Cl.: C07D 311/20

(54) **Process for producing octahydrocoumarin or derivative thereof**
Verfahren zur Herstellung von Octahydrocumarin oder Derivat davon
Procédé de production d'octahydrocoumarine ou son dérivé

(30) Priority: 20.09.1991 JP 240076/91
(43) Date of publication of application: 24.03.1993
(73) Proprietor: SUMITOMO CHEMICAL COMPANY LIMITED, Osaka-shi, Osaka 541 (JP)
(72) Inventor: Sakai, Kiyomi, Niihama-shi, Ehime (JP); Nishida, Yoshitaka, Niihama-shi, Ehime (JP); Shirafuji, Tamio, Niihama-shi, Ehime (JP)
(74) Representative: Dixon, Donald Cossar

(56) References cited:
- JOURNAL OF THE CHEMICAL SOCIETY, PERKIN TRANSACTIONS 2, no. 4, April 1988, Letchworth, GB, pages 431 - 436 D.V. GRIFFITHS ET AL. 'Synthetic and stereochemical studies of the octahydro-1-benzopyran system'

## Description

The present invention relates to a process for producing octahydrocoumarin or a derivative thereof. Octahydrocoumarin and its derivatives are important compounds in the perfume industry and are also useful as intermediates in the chemical industry.

Conventionally known methods for producing octahydrocoumarin or a derivative thereof include, for example, a process comprising reducing coumarin or a derivative thereof with hydrogen (as described in Yakugaku Zasshi (Pharmaceutical Journal), vol. 74, 895-898 (1954)), a process comprising reducing 3-(2-oxocyclohexyl)propionic acid or a derivative thereof with hydrogen (as described in J. Am. Chem. Soc., vol. 73, 724-730 (1951)), a process comprising reducing 3-(2-oxocyclohexyl)propionic acid or a derivative thereof with sodium boron hydride or the like (as described in J. Org. Chem., vol. 27, 4141-4146 (1962)), and a process comprising reacting a hydroxynitrile with the aid of a ruthenium catalyst (as described in JP-A-63-216879). (The term "JP-A" as used herein means an unexamined published Japanese patent application.)

However, these conventional processes are unsatisfactory from an industrial standpoint.
Thus, the reduction of coumarin or its derivative with hydrogen is disadvantageous in that the coumarin or its derivative used as a starting material is expensive and that the reduction with hydrogen necessitates a high hydrogen pressure exceeding 100 atm a considerably large amount. The reduction of 3-(2-oxocyclohexyl)propionic acid or its derivative with hydrogen, sodium boron hydride or the like is not industrially preferable in that the attainable yield of the desired compound is insufficient, and that sodium boron hydride is expensive. Further, the reaction of a hydroxynitrile with the aid of a ruthenium catalyst is disadvantageous in that the hydroxynitrile used as a starting material is expensive.

Document D1 (Journal of the Chemical Society, Perkin Transactions 2, n°4, April 1988, Letchworth, GB, pages 431-438 discloses, on page 432, column 1, third and fourth paragraphs, that 2-(2-(ethoxycarbonyl)ethyl)-cyclohexanone is subjected to catalytic hydrogenation to produce cyclohexanol, which corresponds to the step of hydrogenation of 3-(2-oxocyclohexyl)propionic acid ester or its derivative in the present invention.

Document D1 discloses, as a catalyst of the catalytic hydrogenations Pd(C) (scheme on page 432) and platinum black (page 435, column 2, third and fourth paragraphs), and shows specific reaction conditions and results only for the case of platinum black (page 435, column 2, lines 26-29). According to the results, even if a long reaction time (17 hours) is employed for catalytic hydrogenation of 2-(2-(ethoxycarbonyl)ethyl)cyclohexanone (corresponding to the keto ester in the present invention), 10% of the starting 2-(2-(ethoxycarbonyl)ethyl)cyclohexanone remains in the system.

The present inventors have conducted intensive studies in order to develop an industrially satisfactory process for producing octahydrocoumarin or a derivative thereof. As a result, it has been found that octahydrocoumarin or a derivative thereof can be obtained in good yield if the desired compound is produced by reducing a 3-(2-oxocyclohexyl)propionic acid ester or a derivative thereof with hydrogen and then cyclizing the resulting reaction product, i.e., a 3-(2-hydroxycyclohexyl)propionic acid ester or its derivative with elimination of an alcohol. The present invention is based on this finding.

The present invention provides a process for producing octahydrocoumarin or a derivative thereof represented by formula (3): 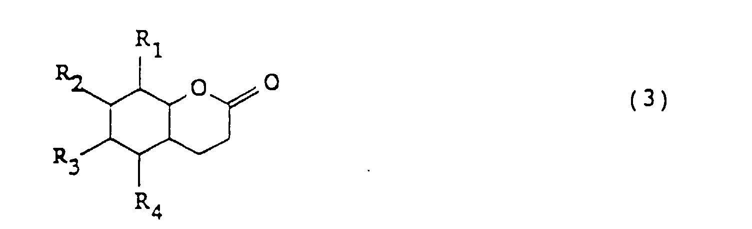 wherein R₁ to R₄ each represents a hydrogen atom or an alkyl group having from 1 to 5 carbon atoms,
the process comprising the steps of: reducing a 3-(2-oxocyclohexyl)propionic acid ester or derivative thereof represented by formula (1): 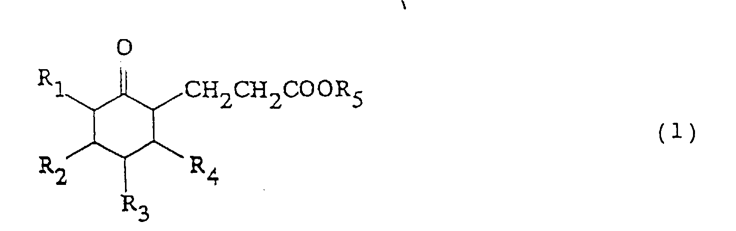 wherein R₁ to R₄ are as defined above and R₅ represents an alkyl group having from 1 to 5 carbon atoms,using hydrogen in the presence of a ruthenium catalyst, thereby to convert the compound of formula (1) to a 3-(2-hydroxycyclohexyl)propionic acid ester or derivative thereof represented by formula (2): 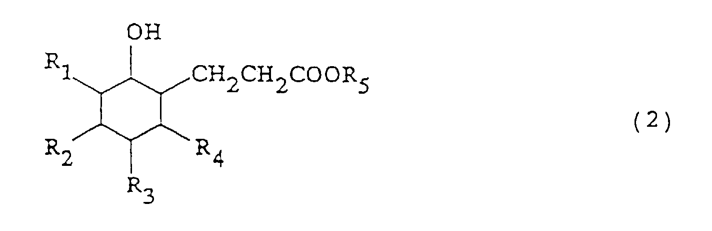 wherein R₁ to R₅ are as defined above; and
then cyclizing the compound of formula (2) thereby to convert the compound of formula (2) to octahydrocoumarin or a derivative with elimination of an alcohol.

Examples of the 3-(2-oxocyclohexyl)propionic acid ester or its derivative used in the present invention, which are represented by formula (1), include
methyl 3-(2-oxocyclohexyl)propionate,
ethyl 3-(2-oxocyclohexyl)propionate,
propyl 3-(2-oxocyclohexyl)propionate,
isopropyl 3-(2-oxocyclohexyl)propionate,
butyl 3-(2-oxocyclohexyl)propionate,
pentyl 3-(2-oxocyclohexyl)propionate,
methyl 3-(3-methyl-2-oxocyclohexyl)propionate,
methyl 3-(5-methyl-2-oxocyclohexyl)propionate,
propyl 3-(4-ethyl-2-oxocyclohexyl)propionate,
methyl 3-(5-isopropyl-2-oxocyclohexyl)propionate,
propyl 3-(4-pentyl-2-oxocyclohexyl)propionate,
propyl 3-(3,4-diethyl-2-oxocyclohexyl)propionate,
propyl 3-(3,4-dimethyl-2-oxocyclohexyl)propionate,
methyl 3-(3,5-diethyl-2-oxocyclohexyl)propionate, and
methyl 3-(3-ethyl-6-methyl-2-oxocyclohexyl)propionate.
However, the compound of formula (1) is not limited to these examples. Among the abode compounds, methyl 3-(2-oxocyclohexyl)propionate and ethyl 3-(2-oxocyclohexyl) propionate are preferably used.

As the catalyst for use in reducing the 3-(2-oxocyclohexyl)propionic acid ester or its derivative with hydrogen, a catalyst generally used for the reduction of ketones with hydrogen is preferably employed. Examples thereof include ruthenium, rhodium, palladium, osmium, iridium, platinum, nickel, copper and chromium. These catalysts may be used either alone or as a mixture of two or more thereof. Particularly preferred of those are ruthenium, rhodium, palladium and platinum.

The catalyst may be one which has been supported on a carrier composed of a Group IIA, IIIA, IVA or VIA element of the periodic table or compounds thereof, such as carbon, alumina, silica gel or barium sulfate. In the case of using such a supported catalyst, the amount of the catalyst metal supported on a carrier is preferably about from 0.1 to 20% by weight based on the total weight of the catalyst. It is possible to use the catalyst together with a compound of molybdenum, zinc or iron. This may be attained by fixing such a compound on a carrier along with the catalyst or otherwise by introducing such a compound into the reaction system. Besides being used as a heterogeneous catalyst, the catalyst may also be used in the form of a salt or a complex of the catalyst metal with a halogen, hydrogen or phosphine.

Although the preferred range of catalyst amount varies depending on the amount of the catalyst supported on a carrier if the catalyst is a heterogeneous one, the amount of the catalyst to be used for the reduction is preferably from 0.01 to 5% by weight as a metal, more preferably from 0.02 to 2% by weight, based on the amount of the 3-(2-oxocyclohexyl)propionic acid ester or its derivative, since too small an amount of the catalyst results in too low a reaction rate, whereas too large an amount thereof not only results in formation of an increased amount of by-products but in an increased cost. The catalyst can be reused.

The reduction with hydrogen may be conducted using a solvent. Examples of the solvent include water, hydrocarbons, halogenated hydrocarbons, alcohols, ethers, organic acids and esters. These solvents may be used either alone or as a mixture of two or more thereof. The solvent occasionally accelerates the reduction. The amount of the solvent is preferably 0.5 to 10 times by weight based on the amount of the 3-(2-oxocyclohexyl) propionic acid ester or its derivative. However, the reduction may be conducted without using a solvent.

For the purpose of accelerating the reduction or for other purposes, an acid or an alkali may be introduced as an additive into the reaction system. Examples of the acid include hydrochloric acid, sulfuric acid, phosphoric acid, and perchloric acid. Examples of the alkali include sodium hydroxide, potassium hydroxide, and ammonia. The amount of the acid or alkali is preferably 0.01 to 10% by weight based on the amount of the 3-(2-oxocylohexyl) propionic acid ester or its derivative.

The reduction is performed at a temperature of generally about from 0 to 250°C, preferably from room temperature to 200°C. Temperatures below about 0°C are not preferred because of low reaction rates, while temperatures exceeding about 250°C are not preferred in that the starting material may be decomposed or other undesired reactions may take place. The partial pressure of hydrogen is preferably about from 0.5 to 200 atm, although it depends on the catalyst. Together with hydrogen, the gas phase may contain an inert gas such as nitrogen, helium, argon or carbon dioxide.

There are cases where in the reduction, part of the reduction product, i.e., a 3-(2-hydroxycyclohexyl)propionic acid ester or a derivative thereof, undergoes cyclization to give octahydrocoumarin or its derivative according to the reaction temperature or other reaction conditions. However, even if the reduction mixture reaction contains both the 3-(2-hydroxycyclohexyl)propionic acid ester or its derivative and the octahydrocoumarin or its derivative thus formed, this does not pose any particular problem in the subsequent step for cyclization and alcohol elimination reaction. The alcohol resulting from the cyclization and alcohol elimination reaction of part of the 3-(2-hydroxycyclohexyl)propionic acid ester or its derivative during the reduction may be removed by distillation or other means during or after the reduction.

In the cyclization and alcohol elimination reaction of the 3-(2-hydroxycyclohexyl)propionic acid ester or its derivative, it is preferred to use an acid or an alkali as a catalyst. Examples of the acid include mineral acids such as hydrochloric acid, sulfuric acid, phosphoric acid and perchloric acid, carboxylic acids such as palmitic acid, stearic acid, phthalic acid and adipic acid, and solid acids such as active carbon, zeolite and acidic ion-exchange resins. Examples of the alkali include sodium hydroxide, potassium hydroxide, sodium carbonate, sodium bicarbonate, amines, and basic ion-exchange resins. The catalyst can be reused.

The amount of the catalyst to be used in the cyclization and alcohol elimination reaction is preferably from 0.01 to 10% by weight based on the amount of the 3-(2-hydroxycyclohexyl)propionic acid ester or its derivative.

This second reaction is generally carried out at a temperature of about from 0 to 300°C, preferably from room temperature to 200°C. Temperatures below about 0°C are not preferred because of low reaction rates, while temperatures exceeding about 300°C are not preferred in that the starting material may be decomposed and other undesired reactions may take place.

It is preferable that the cyclization and alcohol elimination reaction be conducted while either the reaction products, i.e., octahydrocoumarin or its derivative and an alcohol, or only the alcohol is kept being removed from the reaction system by distillation. The removal of the octahydrocoumarin or its derivative and the alcohol by distillation may be effected under a reduced pressure. However, the cyclization and alcohol elimination reaction may be conducted without removing these reaction products.

The octahydrocoumarin or its derivative obtained by the process of the present invention usually is a mixture of the cis and trans isomers. Although this mixture does not usually pose any particular problem, it is possible to use a stereoselective catalyst in the reduction for the purpose of preferentially obtaining either of the isomers or to isolate either of the isomers from the yielded octahydrocoumarin or its derivative.

Each of the cis and trans isomers of the octahydrocoumarin or its derivative obtained by the process of the present invention may be a mixture of optical isomers. Although each mixture does not generally pose any particular problem, it is possible to use a stereoselective catalyst in the reduction for the purpose of preferentially obtaining either of the isomers or to isolate either of the isomers from the yielded octahydrocoumarin or its derivative.

According to the process of the present invention, octahydrocoumarin or a derivative thereof can be produced in good yield by an industrially practicable simple method using an inexpensive starting material.

The present invention will be explained below in more detail with reference to the following examples. Unless otherwise indicated, all percentages in the following examples are by weight.

### EXAMPLE 1

Into a 100-ml autoclave made of SUS (stainless steel) were introduced 25 g of methyl 3-(2-oxocyclohexyl)propionate (having a purity of 95% by weight) and 0.5 g of platinum dioxide catalyst. The inside of the autoclave was pressurized to 15 kg/cm²G with hydrogen, and the contents were then stirred at 1,000 rpm at room temperature (about 25°C) until hydrogen absorption ceased (for about 7 hours), thereby carrying out a reduction.

After completion of the reduction, the catalyst was removed from the reaction mixture by filtration to obtain 20 g of a reduction mixture. Analysis of the thus-obtained reduction mixture by gas chromatography revealed that it contained 71% methyl 3-(2-hydroxycyclohexyl)propionate and 13% octahydrocoumarin. Methyl 3-(2-oxocyclohexyl)propionate was not detected in the reduction mixture.

To the thus-obtained reduction mixture was added 0.03 g of concentrated sulfuric acid. This mixture was subjected to simple distillation at a pressure of 10 mmHg thereby to conduct a cyclization and alcohol elimination reaction. The time period required for the simple distillation was about 1 hour, during which the highest still pot temperature was 145°C. As a result, 13 g of a liquid distillate was obtained. Analysis of the thus-obtained distillate by gas chromatography revealed that it contained 96% octahydrocoumarin. Neither methyl 3-(2-oxocyclohexyl)propionate nor methyl 3-(2-hydroxycyclohexyl)propionate was detected in the distillate. The yield of octahydrocoumarin based on the methyl 3-(2-oxocyclohexyl)propionate used as the starting material was 62%.

### EXAMPLE 2

Into a 100-ml autoclave made of SUS were introduced 12.5 g of methyl 3-(2-oxocyclohexyl)propionate (having a purity of 95% by weight), 0.1 g of a catalyst composed of active carbon and ruthenium supported thereon in an amount of 5% based on the total catalyst, and 10 g of methanol and 2.5 g of water as solvents. The inside of the autoclave was pressurized to 15 kg/cm²G with hydrogen, and the contents were then stirred at 1,000 rpm at a temperature of 50°C for 2.5 hours thereby to conduct a reduction.

After completion of the reduction, the catalyst was removed from the reaction mixture by filtration to obtain 20 g of a reduction mixture. Analysis of the thus-obtained reduction mixture by gas chromatography revealed that the part of the mixture that is exclusive of the solvents contained 77% methyl 3-(2-hydroxycyclohexyl)propionate, 22% octahydrocoumarin, and 0.1% methyl 3-(2-oxocyclohexyl)propionate.

To the thus-obtained reduction mixture was added 0.3 g of 20-wt% aqueous NaOH solution. This mixture was subjected to simple distillation at a pressure of 10 mmHg to conduct a cyclization and alcohol elimination reaction. The time period required for the simple distillation was about 1 hour, during which the highest still pot temperature was 140°C. As a result, 7 g of a liquid distillate was obtained. Analysis of the thus-obtained distillate by gas chromatography revealed that it contained 99% octahydrocoumarin, 0.5% methyl 3-(2-hydroxycyclohexyl)propionate, and 0.1% methyl 3-(2-oxocyclohexyl)propionate. The yield of octahydrocoumarin based on the methyl 3-(2-oxocyclohexyl)propionate used as the starting material was 69%.

### EXAMPLE 3

Into a 100-ml autoclave made of SUS were introduced 12.5 g of methyl 3-(2-oxocyclohexyl)propionate (having a purity of 95% by weight), 0.1 g of a catalyst composed of active carbon and ruthenium supported thereon in an amount of 5% based on the total catalyst, and 10 g of ethanol and 2.5 g of water as solvents. The inside of the autoclave was pressurized to 15 kg/cm²G with hydrogen, and the contents were then stirred at 1,000 rpm at a temperature of 50°C for 2.5 hours thereby to conduct a reduction.

After completion of the reduction, the catalyst was removed from the reaction mixture by filtration to obtain 22 g of a reduction mixture. Analysis of the thus-obtained reduction mixture by gas chromatography revealed that the part of the mixture that is exclusive of the solvents contained 80% methyl 3-(2-hydroxycyclohexyl)propionate, 17% octahydrocoumarin, and 0.1% methyl 3-(2-oxocyclohexyl)propionate.

To the thus-obtained reduction mixture was added 0.1 g of palmitic acid. This mixture was subjected to simple distillation at a pressure of 10 mmHg to conduct a cyclization reaction. The time period required for the simple distillation was about 1 hour, during which the highest still pot temperature was 140°C. As a result, 8 g of a liquid distillate was obtained. Analysis of the thus-obtained distillate by gas chromatography revealed that it contained 68% octahydrocoumarin, 31% methyl 3-(2-hydroxycyclohexyl)propionate, and 0.1% methyl 3-(2-oxocyclohexyl)propionate. The yield of octahydrocoumarin based on the methyl 3-(2-cyclohexanoyl)propionate used as the starting material was 54%.

### EXAMPLE 4

Into a 500-ml autoclave made of SUS were introduced 62.5 g of methyl 3-(2-oxocyclohexyl)propionate (having a purity of 95% by weight), 0.6 g of a catalyst composed of active carbon and ruthenium supported thereon in an amount of 5% based on the total catalyst, and 50.8 g of methanol and 12.6 g of water as solvents. The inside of the autoclave was pressurized to 15 kg/cm²G with hydrogen, and the contents were then stirred at 1,000 rpm at a temperature of 100°C for 2.5 hours thereby to conduct a reduction.

After completion of the reduction, the resulting reaction mixture was filtered to recover 1.4 g of the wet catalyst and to obtain 116 g of a reduction mixture. Analysis of the thus-obtained reduction mixture by gas chromatography revealed that the part of the mixture that is exclusive of the solvents contained 51% methyl 3-(2-hydroxycyclohexyl)propionate and 45% octahydrocoumarin. Methyl 3-(2-oxocyclohexyl)propionate was not detected in the reduction mixture.

To the thus-obtained reduction mixture was added 0.3 g of 20-wt% aqueous NaOH solution. The solvents were then removed from this mixture by subjecting the mixture to simple distillation at ordinary pressure. Subsequently, the resultant liquid was refluxed for 30 minutes and then subjected to simple distillation at a pressure of 10 mmHg to conduct a cyclization and alcohol elimination reaction. The time period required for this simple distillation was about 1 hour, during which the highest still pot temperature was 150°C. As a result, 45 g of a liquid distillate and 1.6 g of a still residue were obtained. Analysis of the thus-obtained distillate by gas chromatography revealed that it contained 99% octahydrocoumarin and 0.1% methyl 3-(2-hydroxycyclohexyl)propionate. Methyl 3-(2-oxocyclohexyl)propionate was not detected in the distillate. The yield of octahydrocoumarin based on the methyl 3-(2-oxocyclohexyl)propionate used as the starting material was 90%.

### EXAMPLE 5

Into a 500-ml autoclave made of SUS were introduced 62.2 g of methyl 3-(2-oxocyclohexyl)propionate (having a purity of 95% by weight), 1.4 g of the catalyst recovered in Example 5, and 50.2 g of methanol and 12.6 g of water as solvents. The inside of the autoclave was pressurized to 15 kg/cm²G with hydrogen, and the contents were then stirred at 1,000 rpm at a temperature of 100°C for 5 hours thereby to conduct a reduction.

After completion of the reduction, the resulting reaction mixture was filtered to obtain 115 g of a reduction mixture. Analysis of the thus-obtained reduction mixture by gas chromatography revealed that the part of the mixture that is exclusive of the solvents contained 80% methyl 3-(2-hydroxycyclohexyl)propionate, 19% octahydrocoumarin, and 1.0% methyl 3-(2-oxocyclohexyl)propionate.

To the thus-obtained reduction mixture was added 1.6 g of the still residue obtained in Example 5. The solvents were then removed from this mixture by subjecting the mixture to simple distillation at ordinary pressure. Subsequently, the resultant liquid was refluxed for 30 minutes and then subjected to simple distillation at a pressure of 10 mmHg to conduct a cyclization and alcohol elimination reaction. The time period required for this simple distillation was about 1 hour, during which the highest still pot temperature was 155°C. As a result, 42 g of a liquid distillate was obtained. Analysis of the thus-obtained distillate by gas chromatography revealed that it contained 99% octahydrocoumarin, 0.01% methyl 3-(2-hydroxycyclohexyl)propionate, and 0.8% methyl 3-(2-oxocyclohexyl)propionate. The yield of octahydrocoumarin based on the methyl 3-(2-oxocyclohexyl)propionate used as the starting material was 84%.

## Claims

1. A process for producing octahydrocoumarin or a derivative thereof represented by formula (3): 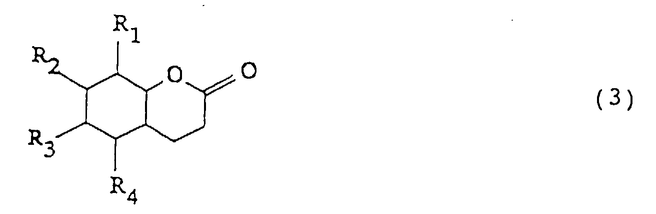 wherein R₁ to R₄ each represents a hydrogen atom or an alkyl group having from 1 to 5 carbon atoms,
said process comprising the steps of: reducing a 3-(2-oxocyclohexyl)propionic acid ester or derivative thereof represented by formula (1): 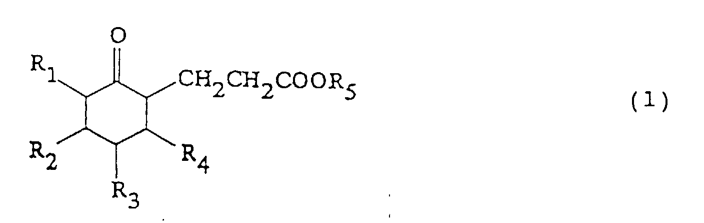 wherein R₁ to R₄ are as defined above and R₅ represents an alkyl group having from 1 to 5 carbon atoms,using hydrogen in the presence of a ruthenium catalyst, thereby to convert the compound represented by formula (1) to a 3-(2-hydroxycyclohexyl)propionic acid ester or derivative thereof represented by formula (2): 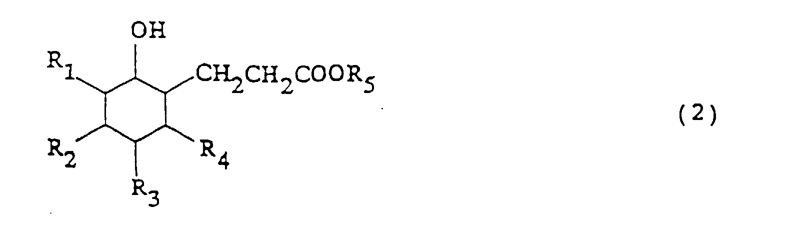 wherein R₁ to R₅ are as defined above; and
then cyclizing the compound represented by formula (2) thereby to convert the compound represented by formula (2) to octahydrocoumarin or derivative with elimination of an alcohol.

2. A process as claimed in Claim 1, wherein said catalyst is used in an amount of from 0.01 to 5% by weight as a metal based on the amount of the 3-(2-oxocyclohexyl)propionic acid ester or derivative thereof.

3. A process as claimed in Claim 1 or 2, wherein the reduction is conducted at a partial pressure of hydrogen of from 0.5 to 200 atm.

4. A process as claimed in any preceding claim, wherein the reduction is conducted at a temperature of from 0 to 250°C.

5. A process as claimed in any preceding claim, wherein the cyclization and alcohol elimination reaction is conducted in the presence of an acid or an alkali.

6. A process as claimed in Claim 5, wherein said acid or alkali is used in an amount of from 0.01 to 10% by weight based on the amount of the 3-(2-hydroxycylohexyl)propionic acid ester or derivative thereof.

7. A process as claimed in any preceding claim, wherein the cyclization and alcohol elimination reaction is conducted at a temperature of from 0 to 300°C.

8. A process as claimed in any preceding claim, wherein said 3-(2-oxocyclohexyl)propionic acid ester or derivative thereof is methyl 3-(2-oxocyclohexyl)propionate or ethyl 3-(2-oxocyclohexyl)propionate.

## Patentansprüche

1. Verfahren zur Herstellung von Octahydrocumarin oder eines Derivats davon der Formel (3): 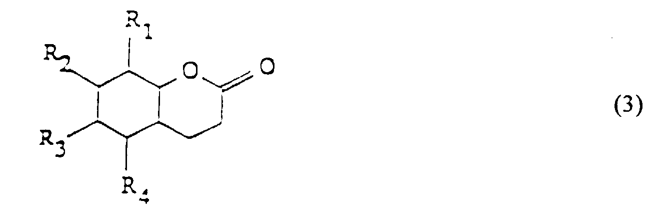 in der R₁ bis R₄ jeweils ein Wasserstoffatom oder einen Alkylrest mit 1 bis 5 Kohlenstoffatomen bedeuten, wobei das Verfahren die Schritte umfaßt: Reduzieren eines 3-(2-Oxocyclohexyl)propionsäureesters oder eines Derivats davon der Formel (1): 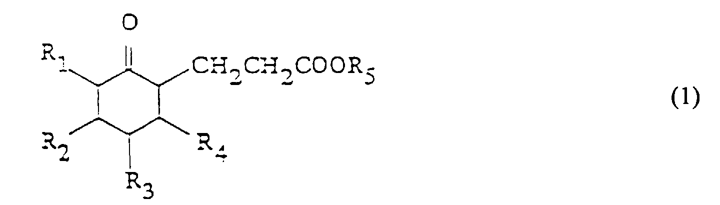 in der R₁ bis R₄ wie vorstehend definiert sind, und R₅ einen Alkylrest mit 1 bis 5 Kohlenstoffatomen bedeutet, unter Verwendung von Wasserstoff in Gegenwart eines Rutheniumkatalysators, wodurch die Verbindung der Formel (1) in einen 3-(2-Hydroxycyclohexyl)propionsäureester oder ein Derivat davon der Formel (2): 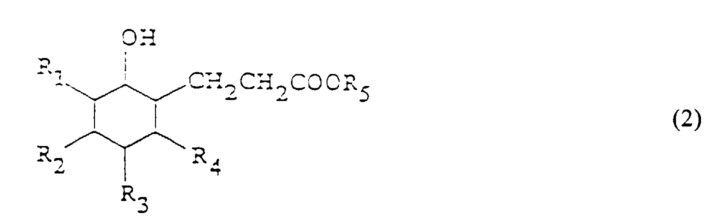 in der R₁ bis R₅ wie vorstehend definiert sind, umgewandelt wird; und anschließend Cyclisieren der Verbindung der Formel (2), wodurch die Verbindung der Formel (2) unter Abspaltung eines Alkohols in Octahydrocumarin oder ein Derivat davon umgewandelt wird.

2. Verfahren nach Anspruch 1, wobei der Katalysator in einer Menge von 0,01 bis 5 Gew.-% als Metall, basierend auf der Menge des 3-(2-Oxocyclohexyl)propionsäureesters oder eines Derivats davon, verwendet wird.

3. Verfahren nach Anspruch 1 oder 2, wobei die Reduktion bei einem Wasserstoffpartialdruck von 0,5 bis 200 atm durchgeführt wird.

4. Verfahren nach einem vorstehenden Anspruch, wobei die Reduktion bei einer Temperatur von 0 bis 250°C durchgeführt wird.

5. Verfahren nach einem vorstehenden Anspruch, wobei die Cyclisierung und die Abspaltung von Alkohol in Gegenwart einer Säure oder eines Alkalis durchgeführt wird.

6. Verfahren nach Anspruch 5, wobei die Säure oder das Alkali in einer Menge von 0,01 bis 10 Gew.-%, basierend auf der Menge des 3-(2-Hydroxycyclohexyl)propionsäureesters oder eines Derivats davon, verwendet wird.

7. Verfahren nach einem vorstehenden Anspruch, wobei die Cyclisierung und die Abspaltung von Alkohol bei einer Temperatur von 0 bis 300°C durchgeführt wird.

8. Verfahren nach einem vorstehenden Anspruch, wobei der 3-(2-Oxocyclohexyl)propionsäureester oder das Derivat davon der 3-(2-Oxocyclohexyl)propionsäuremethylester oder der 3-(2-Oxocyclohexyl)propionsäureethylester ist.

## Revendications

1. Procédé pour produire l'octahydrocoumarine ou un de ses dérivés, composé représenté par la formule (3) : 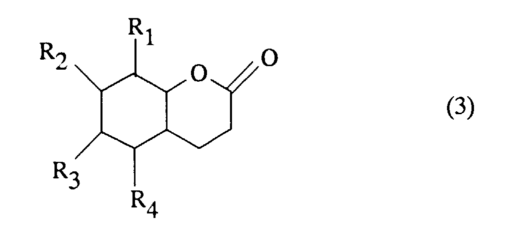 dans laquelle les symboles R₁ à R₄ représentent chacun un atome d'hydrogène ou un groupe alkyle ayant 1 à 5 atomes de carbone, ledit procédé comprenant les étapes consistant à : réduire un ester d'acide 3-(2-oxocyclohexyl)propionique ou son dérivé, composé représenté par la formule (1): 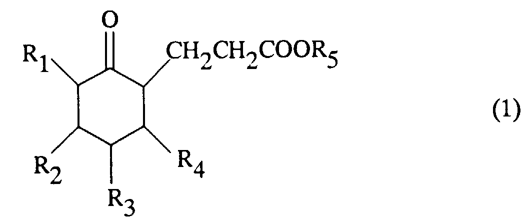 dans laquelle les symboles R₁ à R₄ sont tels que définis ci-dessus, et R₅ représente un groupe alkyle ayant 1 à 5 atomes de carbone, en utilisant l'hydrogène en présence d'un catalyseur au ruthénium, de façon à convertir le composé de formule (1) en un ester de l'acide 3-(2-hydroxycyclohexyl)propionique ou son dérivé, représenté par la formule (2) : 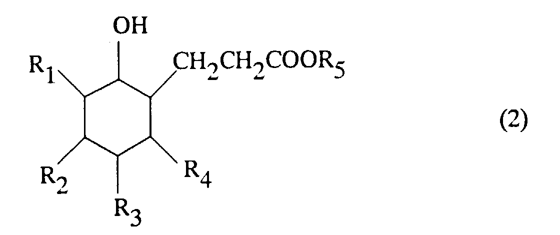 dans laquelle les symboles R₁ à R₅ sont tels que définis ci-dessus ; et
à cycliser ensuite le composé représenté par la formule (2), afin de convertir le composé de formule (2) en de l'octahydrocoumarine ou en son dérivé, avec élimination d'un alcool.

2. Procédé selon la revendication 1, dans lequel on utilise ledit catalyseur en une quantité de 0,01 à 5% en poids, en métal sur la base de la quantité de l'ester d'acide 3-(2-oxocyclohexyl)propionique ou de son dérivé.

3. Procédé selon la revendication 1 ou 2, dans lequel la réduction est conduite à une pression partielle d'hydrogène de 0,5 à 200 at.

4. Procédé tel que revendiqué dans l'une quelconque des revendications précédentes, dans lequel la réduction est conduite à une température de 0 à 250°C.

5. Procédé tel que revendiqué dans l'une quelconque des revendications précédentes, dans lequel la réaction de cyclisation et d'élimination d'alcool est conduite en présence d'un acide ou d'une substance alcaline.

6. Procédé tel que revendiqué à la revendication 5, dans lequel on utilise ledit acide ou ladite substance alcaline en une quantité de 0,01 à 10% en poids, sur la base de la quantité de l'ester d'acide 3-(2-hydroxycyclohexyl)propionique ou de son dérivé.

7. Procédé tel que revendiqué dans l'une quelconque des revendications précédentes, dans lequel on conduit la réaction de cyclisation et l'élimination d'alcool en opérant à une température de 0 à 300°C.

8. Procédé tel que revendiqué dans l'une quelconque des revendications précédentes, dans lequel ledit ester d'acide 3-(2-oxocyclohexyl)propionique ou son dérivé est le 3-(2-oxocyclohexyl)propionate de méthyle ou le 3-(2-oxocyclohexyl)propionate d'éthyle.
